# EUROPEAN PATENT SPECIFICATION

(11) **EP 2 200 535 B1**
(45) Date of publication and mention of the grant of the patent: **09.07.2014**
(21) Application number: 08840564.2
(22) Date of filing: 16.10.2008
(51) Int. Cl.: A61F 2/91, A61F 2/00, A61F 2/02, A61B 17/80, A61B 17/00, A61F 2/08, A61F 2/30

(54) **MEDICAL SHEET**
MEDIZINISCHE FOLIE
FEUILLE MÉDICALE

(30) Priority: 16.10.2007 US 873060
(43) Date of publication of application: 30.06.2010
(73) Proprietor: Cordis Corporation, Miami Lakes, FL 33014 (US)
(72) Inventor: LUEHRS, Kirsten F., Palo Alto California 94306-0000 (US); SILVER, James H., Palo Alto California 94306 (US)
(74) Representative: Belcher, Simon James
(86) International application number: PCT/US2008/080203
(87) International publication number: WO 2009/052309

(56) References cited:
- EP-A- 1 157 673
- EP-A- 1 523 959
- WO-A-2007/087146
- WO-A2-2008/124673
- US-A- 5 776 195
- US-A1- 2002 143 386
- US-A1- 2004 143 154
- US-A1- 2005 172 471

## Description

### FIELD OF THE INVENTION

The present invention relates to a medical sheet made from a super elastic material such as Nitinol for use within a body passageway or duct.

### BACKGROUND OF THE INVENTION

Medical sheets and medical tape are implantable devices and are used to provide structural support within a patient. These support device applications include: slings and organ support sheets, transvascular tape, biological implant materials, fascia and grafts for muscle, tendons, connective tissue, bones, etc. The medical sheets must be flexible and have surface areas that are large enough to provide the necessary support for the internal organ. In order to provide a large flexible surface area, medical sheet and medical tape are frequently a mesh or woven structure.

WO-A-2008/124673, which forms prior art for novelty only under Article 54(3) EPC relates to a tissue repair matrix with a pattern of loops, struts and bridges. Amorphic circles provide rounded surfaces to the outer edges. The tissue repair matrix can be compressed for easier insertion into a patient.

US-A-2002/0143386 describes a medical device in the form of a stent with markers, in the form of rings, formed from housings integral with the stent. The markers have inserts that have a radiopacity which is higher than that of the stent and so increases the visibility under X-ray fluoroscopy

US-A-2004/0143154 discusses a cardiac harness. The harness can comprise spring hinge panels with several strands of spring hinges. An eyelet is disposed at end of each strand at the side edges of each panel. A line of suture material is threaded through the eyelets to connect adjacent panels.

### SUMMARY OF THE INVENTION

The present invention is an improved medical sheet for implantation into a patient. The medical sheet has a first smaller surface area in a compressed state which simplifies the insertion into the patient. After the medical sheet is inserted into the patient it is expanded to a larger surface area in an expanded state. The medical sheet is implanted into the patient in the expanded form.

According to the present invention, there is provided a medical sheet as defined in appended claim 1.

In one exemplary embodiment, the inventive medical sheet is a planar member that is rectangular in shape. Amorphic circles are attached to the outer edges of the medical sheet to remove any sharp points. The rounded amorphic circles make the inventive medical sheet a-traumatic to the implanted patient. The amorphic circles may have holes which allow for in-growth after the medical sheet has been implanted within the patient. Alternatively, the holes in the amorphic circles can be used as suture points that are used to secure the medical sheet to the desired location within the patient.

The medical sheet may be cut into an intricate pattern of interconnected struts, loops and bridges. Bending of the struts, loops and bridges allows the medical sheet to transform in area between a compressed state and an expanded state. After the medical sheet is fabricated, it can be coated with polymers, therapeutic agents, bioactive materials or radio-opaque materials depending upon the application.

The medical sheet is preferably made from a "super elastic" metal alloy such as Nitinol. Super elastic metal alloys have the physical characteristics of being extremely elastic when in the austenitic molecular phase. In this phase, the inventive medical sheet can be compressed into a small volume without springing back to its expanded shape. In the compressed form, the gaps separating the struts, loops and bridges are very small so that the adjacent struts are in very close proximity to each other. In addition to being compressed in a planar manner, the medical sheet may also be bent or rolled out of plane in an accordion manner to further compresses the medical sheet.

Before the medical sheet is implanted in the desired area within the patient, it is heated to change the super elastic alloy to the austenitic phase causing the medical sheet to expand. The final austenite transition temperature may be about 24° C to about 37° C so that exposure to the patient's body heat causes the desired transformation. In the expanded state, the struts, loops and bridges bend so the medical sheet expands to a larger area with larger gaps between the components. After the medical sheet is fully expanded, it has a sufficient area to provide support to the internal organs and can be attached to the patient through in-growth or sutures through the holes in the amorphic circles.

### BRIEF DESCRIPTION OF DRAWINGS

The foregoing and other aspects of the present invention will best be appreciated with reference to the detailed description of the invention in conjunction with the accompanying drawings, wherein:
FIG. 1 is a view of an embodiment of the medical sheet in the compressed state.
FIG. 2 is a view of an embodiment of the medical sheet in the compressed state.
FIG. 3 is an enlarged view of the medical sheet in the compressed state.
FIG. 4 is a side view of an embodiment of the medical sheet folded out of plane.
FIG. 5 is an enlarged view of a portion of the medical sheet in the expanded state.
FIG. 6 is an alternative embodiment of the medical sheet.

### DETAILED DESCRIPTION

The present invention is directed towards both a medical sheet and medical tape which only differ in geometry. The medical sheet is rectangular in shape while the medical tape is a thinner and more elongated structure. For simplicity, the present invention is primarily described as a medical sheet. It is intended for the disclosure to be equally applicable to the medical tape embodiment.

To manufacture the inventive medical sheet, a super elastic metal alloy is required. Although the super elastic alloy is described as Nitinol (Ni-Ti alloy), any alloy with similar super elastic properties may be used. Nitinol sheet stock is commercially available from a number of suppliers including Nitinol Devices & Components, Fremont California. Alternatively, the Nitinol sheet stock may be formed through a deposition process such as chemical vapor deposition, physical vapor deposition, and plasma spray deposition. The deposition process builds a layer of material by depositing molecules of the alloy onto a substrate in a vacuum chamber and then the deposited layer is separated from the substrate. The substrate can have various three dimensional shapes, so the vapor deposited Nitinol can also be formed into a planar or three dimensional layer. The thickness of the super elastic alloy medical sheet may range from about 0.0001 to about 0.1 inch.

The Nitinol sheet stock may be cut into the desired fully expanded medical sheet pattern while in the austenitic phase or in the martensitic phase. The phase of the Nitinol material is temperature dependent. In general, the austenitic transition temperature A_{f} is about 24° C to about 37° C. At temperatures above the austenitic transition temperature, the Nitinol will be in the Austenitic phase. At lower temperatures, the Nitinol may be fully or partially in the martensitic phase. If the medical sheet is cut in the martensitic phase, it can then be maintained in the expanded shape while it is heat treated to convert the Nitinol to the austenitic phase. The medical sheet can then be cooled to transform the medical sheet to the martensitic phase before the medical sheet is compressed into a compact state for implantation in a patient. The austenitic phase shape is the shape that the medical sheet will attempt to assume whenever it is heated above the austenitic transition temperature.

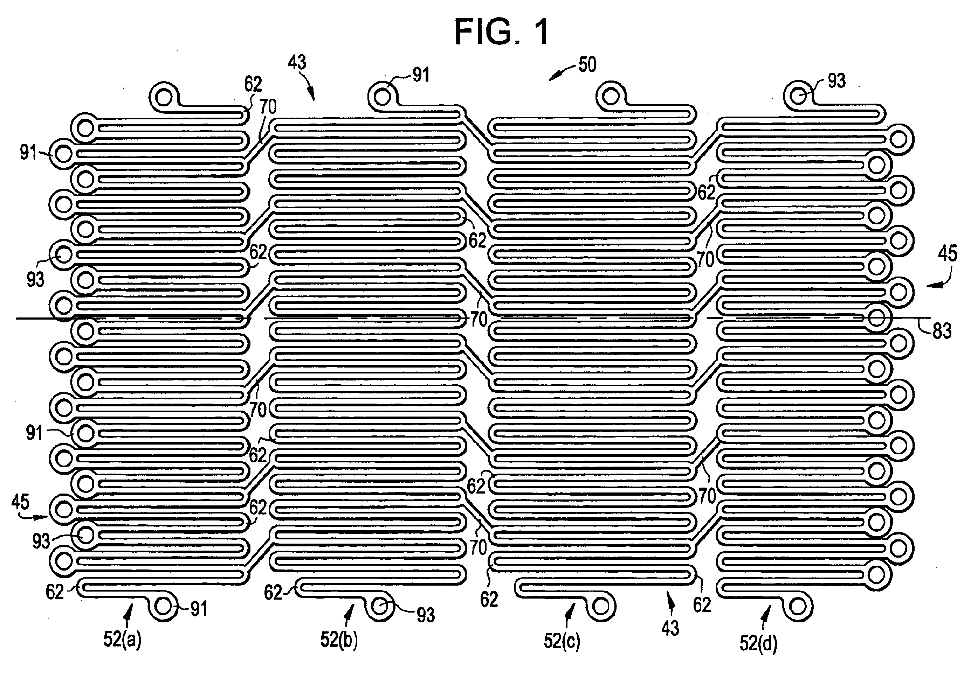

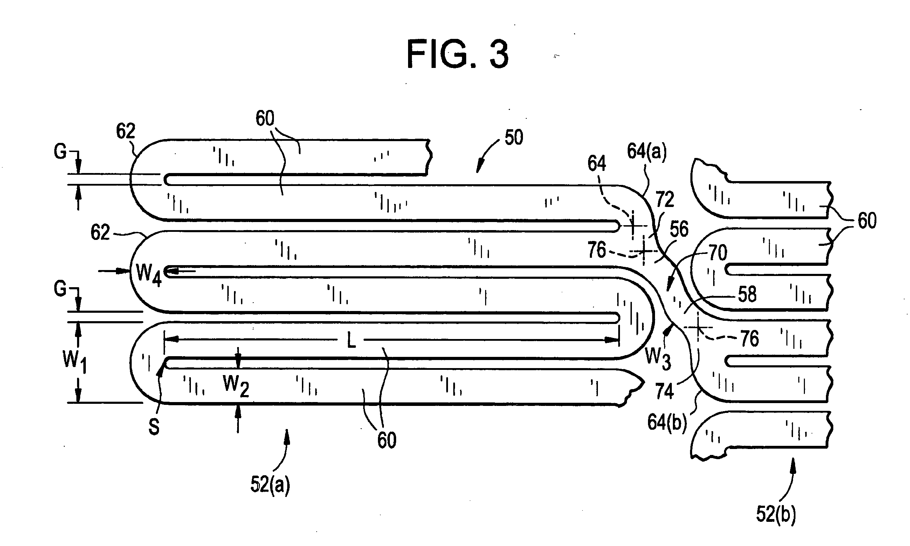

With reference to FIG. 1 and FIG. 3, the medical sheet 50 is cut into an intricate pattern of adjacent planar strips 52(a)-52(d) that are connected by a plurality of bridges 70. The planar strips 52(a)-52(d) are each made of a plurality of interconnected struts 60 and loops 62. The lengths of the struts, loops and bridges may vary within each of the planar strips 52(a)-52(d). In an embodiment, the Nitinol sheet stock is loaded into a machine that cuts the predetermined pattern of the expandable medical sheet. Machines that can cut sheets of Nitinol are well known to those of ordinary skill in the art and are commercially available. During this machining process, the metal sheet is typically held stationary while a cutting tool, preferably under microprocessor control, moves over the sheet and cuts the desired medical sheet pattern. The pattern dimensions and styles, laser positioning requirements, and other information are programmed into a microprocessor which controls all aspects of the process. The cutting tool can be a laser, laser chemical etch, water jet, electrical discharge machining, etc.

In an exemplary embodiment, a photochemical etch process may be used to cut the desired pattern into the Nitinol sheet. This process can include various process steps that are generally known as photolithography. A photoresist layer is deposited onto the Nitinol sheet and exposing the photosensitive layer to a pattern of light that matches the desired pattern that the sheet is to be cut into. The light chemically alters the exposed areas of the photoresist layer and a chemical reaction is used to remove the portions of the photosensitive layer that were not exposed to light. An etch process then cuts through the areas of the Nitinol that are not covered by the photoresist to form the patterned medical sheet. The remaining photoresist is removed to produce the finished patterned medical sheet.

In an embodiment, the medical sheet 50 may be made from a plurality of adjacent elongated planar strips 52(a)-52(d) that are secured adjacent to each other across the length of the medical sheet 50 by a plurality of bridges 70. Although four adjacent elongated strips are shown, medical sheets with any number of elongated strips can be made. The edges of the medical sheet 50 that are formed by the ends of the elongated strips 52(a)-52(d) are the retractable sides 43 of the medical sheet 50. The edges of the medical sheet 50 that are formed by the left side of the elongated strip 52(a) and the right side of elongated strip 52(d) are the expandable sides 45 of the medical sheet 50. A longitudinal axis 83 extends between the retractable sides 43 of the medial sheet 50.

The elongated strips 52 each include a plurality of longitudinal struts 60 and a plurality of loops 62 that connect the adjacent struts 60. The adjacent struts 60 are connected with loops 62 in an alternating pattern at opposite ends of the struts 62, forming a serpentine or "S" shaped pattern. In the compressed state, the loops 62 are substantially semi-circular and appear to be about a 180° bend. The space between the struts 60 is very small because the 180° bends of the loops 62 cause the struts 60 to be compressed close to each other.

The medical sheet also includes a plurality of amorphic circles 91 that are attached along the outer edges of the medical sheet 50. Along the short sides of the medical sheet 50, the amorphic circles 91 are attached to the loops 62. Along the long sides of the medical sheet 50, the amorphic circles 91 are attached at a terminal point along the longitudinal length of the outermost strut in the planar strip 52(a) - 52(d). The rounded surfaces of the amorphic circles 91 and loops 62 eliminate any sharp external features and make the medical sheet 50 a-traumatic when implanted into a patient. The Amorphic circles 91 are round features that may also have a smooth rounded edge, such as a "bull-nose" edge to further remove any sharp surfaces. The amorphic circles 91 will preferably have a rounded ring type shape rather than a cylindrical shape with sharp edges. The radius of the rounded edges will be less than one half the thickness of the Nitinol sheet and preferably less than one quarter of the thickness of the sheet.

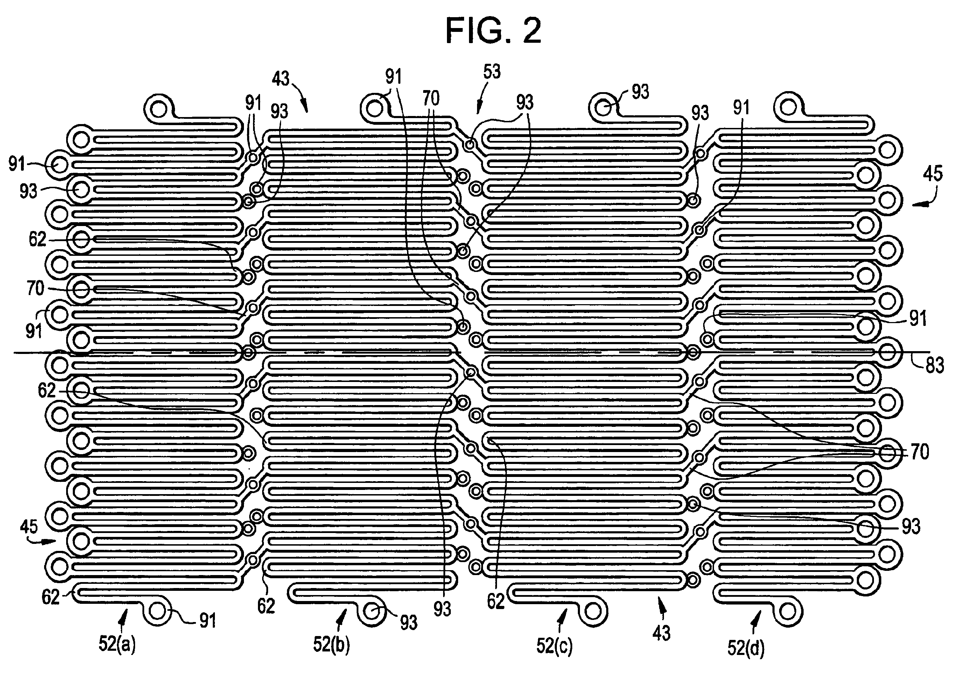

Holes 93 in the amorphic circles 91 provide areas for ingrowth to stabilize the medical sheet 50 implanted into a patient. Alternatively, the holes 93 in the amorphic circles 91 may also be used to suture the medical sheet 50 to tissue within the patient. The medical sheet may be used as a physical graft structure or to provide physical support to organs within a patient. The amorphic circles 91 can range in diameters from about 0.001 to about 0.250 inch. The holes 93 are concentric with the amorphic circles 91 and may be proportional in diameter. The diameters of the holes 93 may range from about 10% to about 90% of the diameter of the amorphic circle 91. Although the amorphic circles 91 and holes 93 are illustrated only around the perimeter, the amorphic circles 91 can also be attached to any interior loop 62 of the medical sheet 53 as shown in FIG. 2.

FIG. 2 illustrates an embodiment of the medical sheet 53 having amorphic circles 91 and holes 93 placed in the bridges 70 and loops between the planar strips 52(a) - 52(d). This interior amorphic circles 91 and holes 93 provide additional areas for ingrowth and suture points to secure the medical sheet within the patient. The internal amorphic circles 91 also allow the medical sheet 53 to be cut to a size that is appropriate for the application. The bridges 70 can be cut to remove one or more of the elongated strips 52(a) - 52(d) from the medical sheet 53. By cutting the bridges 70 next to the amorphic circles 91, the cut medical sheet 53 has amorphic circles 91 that allows the cut edge to be sutured within the patient. The cut bridges 70 may need to be further smoothed after being cut to remove any sharp surfaces.

In alternative embodiments, some of the amorphic circles 91 may be replaced with rounded structures that provide the same a-traumatic edges to the medical sheet 50 as the amorphic circles 91. These rounded structures may be spheres, ovals, rounded rectangles, rounded triangles, a rounded "T" end, etc. Like the amorphic circles 91, these rounded structures can be attached to the loops 62 anywhere in the medical sheet 50, within any of the bridges 70 or at the struts 60 at the ends of the elongated strips 52(a)-52(d). The rounded structures may also have holes formed through their centers for ingrowth or sutures.

Although the bridges 70 appear to be straight structures connected to loops 62 on adjacent planar strips at an angle as shown in FIGS. 1 and 2, the bridges 70 may be curved to improve the structural performance of the inventive medical sheet 50. The bridges 70 can best be described by referring to FIG. 3 that is an enlarged view of a portion of an embodiment of the compressed medical sheet 50. Each bridge 70 has ends 56 and 58. End 56 of bridge 70 is attached to one loop 62 at a bridge-to-loop connection point 72 on a first elongated strip 52(a) and another end 58 attached to another loop 62 at a bride-to-loop connection point 74 on an adjacent elongated strip 52(b), In this example, the end 56 of bridge 70 is connected to loop 64(a) at bridge-to-loop connection point 72 and end 58 is connected to loop 64(b) at bridge-to-loop connection point 74. The bridge-to-loop connection points 72, 74 are separated angularly with respect to the longitudinal axis 83 of the medical sheet 50 and are not horizontally opposite from each other.

The geometry of the struts is also designed to better distribute strain throughout the medical sheet and minimize the opening size between the struts, loops and bridges. The number of struts, loops and bridges as well as the design of these components are important factors when determining the working properties and fatigue life properties of the medical sheet. A medical sheet that has a larger quantity of smaller sized struts per elongated strip improves the mechanical properties of the medical sheet by providing greater rigidity than sheets made with fewer and larger struts. For example, a medical sheet where the ratio of the number of struts per elongated strip to the strut length L (in inches) that is greater than 400 has increased rigidity.

After the medical sheet is cut to the desired pattern, surface processing can be performed. The medical sheet may be passivated by exposing the Nitinol to oxygen to form a layer of metal oxide which helps to prevent corrosion. The medical sheet may also be polished to remove any rough surfaces through processes such as: mechanical polishing, electro polishing or chemical mechanical polishing. This polishing removes any sharp surfaces that may have been formed during the medical sheet cutting processes.

Alternatively, the medical sheet may be textured to improve the ingrowth after implantation or improve the adhesion of coatings applied to the medical sheet. The texturing can be through photochemical etching, sand blasting, tumbling, etc. These textured surfaces can then be coated with different materials that will improve the implanted performance. These chemical coatings are generally intended to improve the biocompatibility of the medical sheet within the patient's body by enhancing ingrowth, preventing rejection and resisting infection. These surface coatings include polymers, therapeutic agents and bioactive materials.

In an embodiment, some of the medical sheet may also be coated with a radio-opaque material that is detectable with x-rays. The radio-opaque materials may alternatively be attached to the Nitinol medical sheet by laser welding, adhesives, mechanical fasteners, etc. After the medical sheet has been implanted within the patient, the implant area can be x-rayed to determine the exact position of the medical sheet. If the medical sheet is improperly positioned, the error can be detected and corrected.

After the medical sheet 50 is cut and all surface coatings are applied, it is ready for use. The medical sheet 50 is cooled below the martensitic transformation temperature to change the Nitinol to a super elastic material. The martensitic transformation temperature M_{f} may be between about 0° to 15° C. In the martensitic phase, the interconnected struts 60, loops 62 and bridges 70 of the medical sheet 50 can be compressed into a small area as shown in FIGS. 1-3. In the compressed shape, there may be very small gaps G between the adjacent struts 60 and loops 62. The compressed Nitinol alloy will remain in the compressed shape as long as the temperature remains below the austenitic transition temperature.

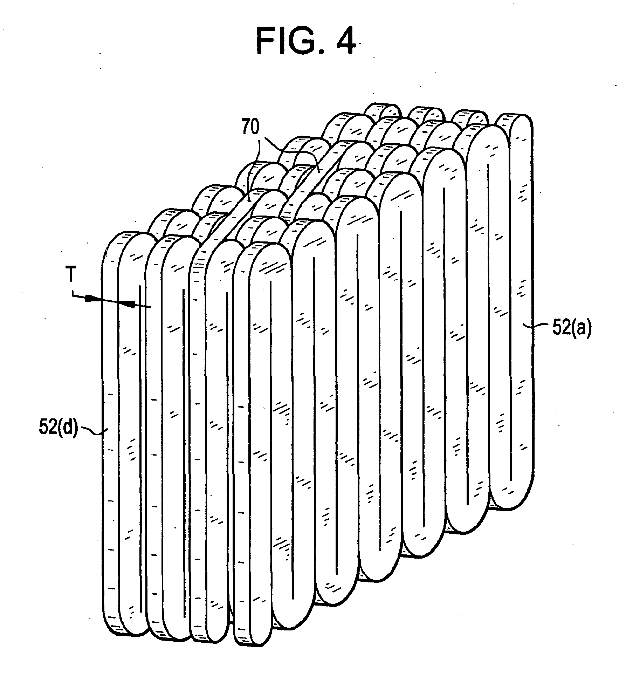

Although the medical sheet 50 is shown in FIGS. 1-3 as being compressed in a planar configuration, it is also possible to further compress the medical sheet 50 out of plane. FIG. 4 shows a side view of the medical sheet 50 that is folded at the bridges 70 in an accordion manner. It is also possible to roll the medical sheet 50 in the compressed state. In the martensitic phase, the medical sheet 50 will retain any of these out of plane compressed shapes until the phase of the metal is changed to the austenitic phase.

To implant the medical sheet 50 into a patient, the compressed medical sheet is held by a delivery apparatus and is inserted through a small incision cut through the skin of the patient. After the medical sheet is inserted inside the patient, it is fully expanded before being permanently or temporarily implanted in the patient. The expansion of the medical sheet inside the patient results from a molecular transformation of the metal alloy from the martensitic phase to the austenitic phase which results from the increased temperature inside the patient's body. The patient's body heat converts the phase of the Nitinol material into the austenitic phase. As the molecular structure of the metal alloy changes to the austenitic phase, the medical sheet decompresses into its expanded shape.

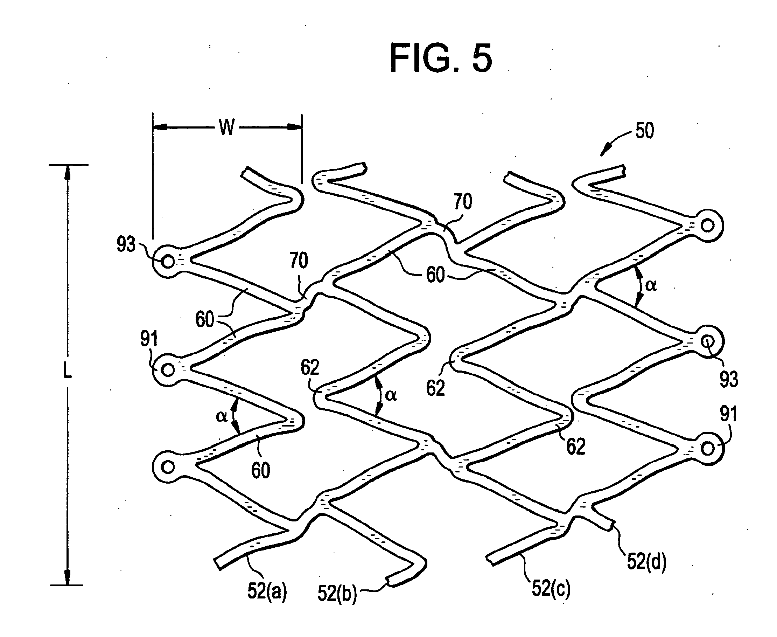

With reference to FIG. 5, a portion of the medical sheet 50 is shown in the austenitic phase and expanded state. The expansion of the medical sheet 50 may only be in line with the lengths of elongated strips 52(a) - 52(d). In the expanded state, the angle α between adjacent struts 60 connected by the loops 62 increases from a compressed angle of about 0° to about 5° to an expanded angle of about 30° to about 70°. The expanded angle α of the loops 62 causes the struts 60 to separate and causes the elongated strips 52(a) - 52(c) to expand in length. As the lengths of the strips expand, the widths of the elongated strips 52(a) - 52(c) get narrower because the struts 60 are angled across the width rather than running perpendicular across the widths. While the medical sheet 50 is shown as being planar in the expanded state, it is possible to build a medical sheet having a three dimensional shape in the expanded state and the compressed state as shown in FIG. 4.

After being fully expanded inside the patient, the medical sheet 50 is positioned and secured in the patient using other medical instruments. The medical sheet 50 may be attached within the patient's body by ingrowth through the holes 93 in the amorphic circles 91 and the gaps G between the struts 60, loops 62 and bridges 70. Alternatively, sutures may be sewn through the holes 93 to secure the medical sheet 50 in place. After the medical sheet 50 is implanted, all surgical tools are removed so the patient can heal.

As seen from FIGS. 1-5, the geometry of the medical sheet changes significantly from the compressed state to its fully expanded state. As the medical sheet expands, the strut angle α and strain levels in the struts, loops and bridges are affected. Preferably, all of the struts, loops and bridges will strain in a predictable manner so that the medical sheet is structurally reliable and uniform in strength. In addition, it is preferable to minimize the maximum strain experienced by struts, loops and bridges, since Nitinol's mechanical strength properties are generally limited by strain rather than by stress like most metal materials. Most metals have a linear relationship between stress and strain in an elastic region and break after the stress exceeds the maximum tensile strength of the metal.

In contrast, when stress is applied to a specimen of a metal such as Nitinol exhibiting super elastic characteristics at a temperature above which the austenite is stable (i.e. the temperature at which the transformation of martensite phase to the austenite phase is complete), the specimen deforms elastically until it reaches a particular stress level where the alloy then undergoes a stress-induced phase transformation from the austenite phase to the martensite phase. As the phase transformation proceeds, the alloy undergoes significant increases in strain but with little or no corresponding increases in stress. The strain increases while the stress remains essentially constant until the transformation of the austenite phase to the martensite phase is complete. Thereafter, further increases in stress are necessary to cause further deformation. The martensitic metal first deforms elastically upon the application of additional stress and then plastically with permanent residual deformation.

If the load on the specimen is removed before any permanent deformation has occurred, the martensitic phase specimen will elastically recover and transform back to the austenite phase. The reduction in stress first causes a decrease in strain. As stress reduction reaches the level at which the martensite phase transforms back into the austenite phase, the stress level in the specimen will remain essentially constant (but substantially less than the constant stress level at which the austenite transforms to the martensite) until the transformation back to the austenite phase is complete, i.e. there is significant recovery in strain with only negligible corresponding stress reduction. The alloys are structurally stronger and more rigid in the austenitic phase than the martensitic phase. After the transformation back to austenite is complete, further stress reduction results in elastic strain reduction. This ability to incur significant strain at relatively constant stress upon the application of a load and to recover from the deformation upon the removal of the load is commonly referred to as super elasticity or "shape memory." See for example, U.S. Pat. No. 4,665,905 (Jervis) and U.S. Pat. No. 4,925,445 (Sakamoto et al.).

The transition between martensite and austenite phases can be controlled by the material temperature. The shape material is fully martensitic when it is colder than the final martensitic transition temperature M*_{f}* and fully austenitic when the material is heated above the final austenitic transition temperature A*_{f}.* The alloy may be partially martensitic and partially austenitic at temperatures between the final martensitic transition temperature M*_{f}* and the final austenitic transition temperature A*_{f}.* These shape memory alloys are stronger in the full austenitic phase than in the martensitic state, but no longer have the super elastic property. When a shape memory alloy structure is heated, it reverts, or attempts to revert, to its original heat-stable shape.

The super elastic metal alloys may comprise nickel, titanium and additional elements such as: niobium, hafnium, tantalum, tungsten and gold. The ratio of the nickel and titanium in the super elastic alloy will alter the martensite/austenite transition temperatures. An alloy having more than 50.5 atomic % nickel has a complete transition temperature from the martensite phase to the austenite phase (A_{f}) below human body temperature, so that austenite is the only stable phase at body temperature. The alloy preferably has an A*_{f}* in the range from about 24° C to about 37° C. The M_{f} is about 25 to about 50 degrees C lower than the A_{f}.

Because these super elastic alloys are capable of extreme deformation, it is desirable to design products that will not exceed the maximum allowable strain during use. In trying to minimize the maximum strain experienced by the struts, loops and bridges, the present invention utilizes a structural geometry that distributes strain to areas of the medical sheet which are less susceptible to failure. For example with reference to FIG. 3, one of the most vulnerable areas of the medical sheet 50 is the inside surface S of the connecting loops 62 defined by the inner radius which undergoes the most deformation and therefore has the highest level of strain of all the medical sheet features. This area is also critical in that it is usually compressed into the smallest radius on the medical sheet. Stress concentrations are minimized by designing the loops 62 with the largest radii possible and/or having a smaller change in angle α between the compressed and fully expanded states shown in FIG. 5.

It is also desirable to minimize local strain concentrations on the bridge 70 and bridge connection points 72, 74. This can be accomplished at the outset through efficient utilization of materials in the struts 60, loops 62 and bridges 70 increases the strength and the ability of the inventive medical sheet 50 to provide structural support. These strain concentrations can also be minimized by utilizing the largest possible curvature radii in the bridges 70 while maintaining feature widths that are proportional to the applied forces. Another way to minimize the strain concentrations is to minimize the maximum open area between the struts 60, loops 62 and bridges 70 in the medical sheet in the expanded state.

These design characteristics are illustrated in FIG. 3. The largest radii curvature features in inventive medical sheet are at the bridge-to-loop connections 76 which are non-symmetric with respect to the centers 64 of the strut connecting loop 62. In other words, the bridge-to-loop connection point centers 76 are off set from the center 64 of the loops 62 to which they are attached. The non-symmetric bridge connection point 76 is particularly advantageous for medical sheets 50 having large expansion ratios because such sheets have extreme bending requirements and large elastic strains.

In a preferred embodiment, there is also a geometric relationship between the widths and the thicknesses of the loops 62 and struts 60. As shown in FIG. 3, the medical sheet 50 has strut connecting loops 62 having a width W4, as measured at the center 64. The widths W4 of the connection loops 62 are greater than the strut 60 widths W2. It is preferable that the thickness of the loops 62 vary so that they are thickest near their centers 64. This increases strain deformation at the strut 60 and reduces the maximum strain levels at the extreme radii of the loop 62. This width-to-thickness relationship reduces the risk of medical sheet failure and maximizes the strength properties. Again, this design feature is particularly advantageous for medical sheets having large expansion ratios and thus extreme bending and large elastic strains.

In a preferred embodiment, there is also a geometric relationship between axial widths of the struts, loops and bridges and the thickness of the medical sheet. With reference to FIG. 3, the axial widths of the struts 60, loops 62 and bridges 70, W2, W4 and W3 (respectively) should be equal or less than the thickness T of the medical sheet 50 shown in FIG. 4. When the medical sheet 50 is in the compressed state, most of the bending occurs in the plane of the medical sheet 50. Therefore, substantially all bending and, therefore, all strains are "out of plane". This minimizes twisting of the medical sheet 50 and minimizes or eliminates buckling and unpredictable strain conditions. This feature is also advantageous for medical sheets having large expansion ratios and thus extreme bending requirements and large elastic strains.

If the bridges, loops and struts have widths that are greater than the material thickness, they have a greater resistance to in-plane bending than out-of-plane bending. In this configuration, the bridges and struts tend to bend out-of-plane, which may cause a buckling condition that is unpredictable and can cause potentially high strain. These problems have been solved by reducing the width of these features to equal or less than the material thickness.

Nitinol can withstand extremely large amounts of elastic strain deformation, so the above features are well suited to medical sheets made from this alloy. This feature allows for maximum utilization of Nitinol or other material capabilities to enhance radial strength, improve medical sheet strength uniformity, improves fatigue life by minimizing local strain levels and improves medical sheet apposition in irregular organ wall shapes and curves.

Another design feature that improves the uniform expansion of the medical sheet is the angle of the bridges that connect the adjacent elongated sections of the inventive medical sheet. As the medical sheet is transformed from its compressed state to its expanded state, strains are applied to the struts and loops. The forces of the expanding struts and loops are delivered to the bridge ends and alter the angle of the bridges with respect to the loops to which they are connected. As shown in FIGS. 1, 2 and 5, the angles of the bridges 70 connecting the first elongated strip 52(a) to the second elongated strip 52(b) and the third elongated strip 52(c) to the fourth elongated strip 52(d) are angled upwardly from left to right in an identical manner but the bridges connecting between the second elongated strip 52(b) and the third elongated strip 52(c) are angled downward from left to right in the opposite direction. This pattern of alternating bridge angles between the elongated strips would continue across a medical sheet having additional elongated strips. This alternating bridge 70 slope pattern improves the rigidity of the medical sheet 50 and minimizes any asymmetric movement or misalignment of the medical sheet 50 within the patient. This symmetric deformation is particularly beneficial if the medical sheet starts to shear in vivo.

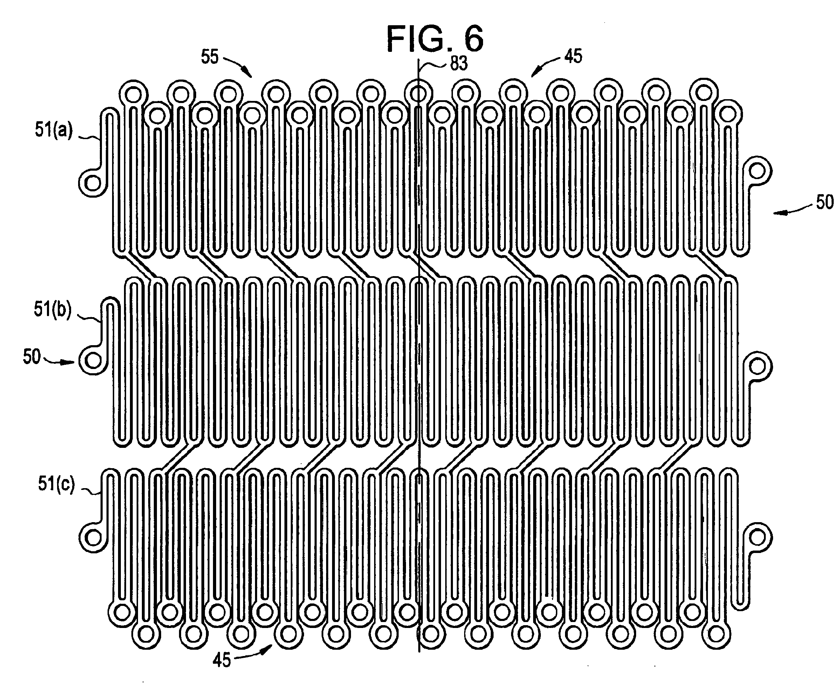

In an alternative embodiment illustrated in FIG. 6, the medical sheet 55 has elongated strips 51(a)-51(c) that run horizontally across the length of the medical sheet 55. The adjacent elongated strips 51 (a)-51 (c) include struts 60 and loops 62 that couple the adjacent struts 60. The adjacent elongated strips 51(a)-51(c) are coupled to each other with bridges 70. The elongated strips 51(a)-51(c) expand horizontally along the length of the medical sheet 55 rather than expanding across the width of the medical sheet as shown in FIGS. 1, 2, and 5. The ends of the elongated strips 52(a)-52(c) form the retractable sides 43 of the medical sheet 55. The edges of the medical sheet 55 that are formed by the upper side of the elongated strip 52(a) and the lower side of elongated strip 52(c) are the expandable sides 45 of the medical sheet 55. A longitudinal axis 83 extends between the retractable sides 43 of the medial sheet 55.

In addition to changing the direction of expansion, the alignment of the elongated strips will also influence the mechanical properties of the medical sheet in the expanded state. With reference to Fig. 5, the repair matrix are more elastic in the expanded state in a direction along the longitudinal direction L of the elongated strips 52(a) - 52(d). In contrast, the elongated strips 52(a) - 52(d) are less elastic across their widths W. Thus, the medical sheet shown in FIGS. 1 and 2 with vertically oriented elongated strips 52(a)-52(d) will be more vertically elastic in the expanded state. Similarly, the medical sheet shown in FIG. 6 has horizontally oriented elongated strips 51(a)-51(c) and will be more horizontally elastic in the expanded state.

According to the description herein, different medical sheets for many implantation applications can be built by altering the lengths and number of elongated sections. The inventive medical sheet can be built for very specific applications including: vaginal slings, fascial slings, suburethral slings, bladder slings and other organ support applications.

Although particular embodiments of the present invention have been shown and described, modification may be made to the device and/or method without departing from the scope of the present invention which is defined by the appended claims.

## Claims

1. A medical sheet (50, 55) for implantation into a patient, the medical sheet comprising;
a) a planar member having a thickness, first and second expandable sides (45), first and second retractable sides (43) and a longitudinal axis (83) extending between the first and the second retractable sides, the planar member having a first smaller area position for insertion into the patient, and a second larger area position for implantation into the patient;
b) the planar member comprising a plurality of adjacent strips (51(a)-51(c), 52(a)-52(d)) extending parallel to each other along the longitudinal axis of the planar member, the strips comprising a plurality of longitudinal struts (60) and a plurality of loops (62) connecting adjacent struts;
c) a plurality of bridges (70) connecting the adjacent strips to one another at bridge-to-loop connection points (72, 74), wherein the bridge-to-loop connection points for each bridge are separated angularly with respect to the longitudinal axis; and
d) a plurality of amorphic circles (91), wherein some of the amorphic circles are attached to at least some of the plurality of loops along the first and second expandable sides (45), and some of the plurality of amorphic circles are attached to some of the plurality of struts along the first and second retractable sides (43) at a terminal point along the longitudinal length of the outermost strut in the adjacent strips; and
wherein the bridges, loops and struts have widths (W2, W3, W4) which are less than the thickness (T) of the planar member.

2. The medical sheet according to claim 1 wherein the loops comprise curved substantially semi-circular sections having centers (64) and the bridge-to-loop connection points are offset from the centers of the loops.

3. A medical sheet according to claim 1, wherein the planar member is made from a super elastic Nickel Titanium alloy, and wherein the number of bridge-to-loop connection points is less than the total number of loops on the strips.

4. The medical sheet according to claim 3 wherein the loops comprise curved substantially semi-circular sections having centers (64), the bridges being connected to the loops at bridge-to-loop connection points having centers (76), the centers of the points are offset from the centers of the loops.

5. The medical sheet according to claim 3 wherein the medical sheet is made from an alloy comprising from about 50.5 percent to about 60 percent Nickel and the remainder comprising Titanium.

6. The medical sheet according to claim 5 wherein the medical sheet has an A_{f} temperature between about 24° to about 37° Celsius.

7. The medical sheet according to claim 1 or 3 wherein at least some of the plurality of amorphic circles have concentric holes (93).

8. A medical sheet according to claim 6 wherein the bridges have a non-linear curved profile between the bridge-to-loop connection points.

9. The medical sheet according to claim 1, 3 or 8 wherein some of the plurality of amorphic circles are attached to some of the plurality of bridges.

10. The medical sheet according to claim 1, 3 or 8 wherein the amorphic circles have rounded edges.

11. The medical sheet according to any one of the preceding claims, wherein some of the amorphic circles (91) are placed in bridges (70) and loops between adjacent strips (52(a)-52(d)).

## Patentansprüche

1. Medizinische Folie (50, 55) zum Implantieren in einem Patienten, wobei die medizinische Folie Folgendes umfasst:
a) ein ebenes Teil mit einer Dicke, erste und zweite sich erstreckende Seiten (45), erste und zweite rückziehbare Seiten (43) und eine Längsachse (83), welche sich zwischen den ersten und zweiten rückziehbaren Seiten erstreckt, wobei das ebene Element eine erste Position eines kleineren Bereichs zum Einführen in den Patienten und die zweite Position eines größeren Bereichs zur Implantation in dem Patienten aufweist;
b) wobei das ebene Element eine Anzahl von angrenzenden Streifen (51 (a)-51 (c), 52(a)-52(d)) umfasst, die sich parallel zueinander entlang der Längsachse des ebenen Elements erstrecken, wobei die Streifen mehrere Längsstreben (60) und mehrere Ösen (62), die angrenzende Streben verbinden, umfassen;
c) mehrere Brücken (70), welche angrenzende Streifen an den Brücken-Ösen-Verbindungspunkten (72, 74) miteinander verbinden, wobei die Brücken-Ösen-Verbindungspunkte für jede Brücke in einem Winkel gegenüber der Längsachse getrennt sind; und
d) mehrere amorphe Kreise (91), wobei einige der amorphen Kreise an wenigstens einigen der mehreren Ösen entlang der ersten und zweiten erweiterbaren Seiten (45) angebracht sind, und einige der mehreren amorphen Kreise an einigen der Streben entlang der ersten und zweiten rückziehbaren Seiten (43) an einem Endpunkt entlang der Längsachse der äußersten Strebe in den angrenzenden Streifen angebracht sind; und
wobei die Brücken, Ösen und Streifen Breiten (W2, W3, W4) aufweisen, die kleiner sind als die Dicke (T) des ebenen Elements.

2. Medizinische Folie nach Anspruch 1, wobei die Ösen gekrümmte, im Wesentlichen halbkreisförmige Abschnitte mit Zentren (64) aufweisen und die Brücken-Ösen-Verbindungspunkte gegenüber den Zentren der Ösen versetzt sind.

3. Medizinische Folie nach Anspruch 1, wobei das ebene Element aus einer superelastischen Nickel-Titan-Legierung gebildet ist und wobei die Anzahl der Brücken-Ösen-Verbindungspunkte geringer ist als die Gesamtanzahl der Ösen auf den Streifen.

4. Medizinische Folie nach Anspruch 3, wobei die Ösen gekrümmte, im Wesentlichen halbkreisförmige Abschnitte mit Zentren (64) umfassen, wobei die Brücken mit den Ösen an Brücken-Ösen-Verbindungspunkten mit Zentren (76) verbunden sind, wobei die Zentren der Punkte gegenüber den Zentren der Ösen versetzt sind.

5. Medizinische Folie nach Anspruch 3, wobei die medizinische Folie aus einer Legierung gebildet ist, die von etwa 50,5% bis etwa 60% Nickel und im Übrigen Titan umfasst.

6. Medizinische Folie nach Anspruch 5, wobei die medizinische Folie eine A_{f}-Temperatur zwischen etwa 24°C bis etwa 37°C aufweist.

7. Medizinische Folie nach Anspruch 1 oder 3, wobei wenigstens eine der mehreren amorphen Kreise konzentrische Löcher (93) aufweisen.

8. Medizinische Folie nach Anspruch 6, wobei die Brücken ein nicht lineares, gekrümmtes Profil zwischen den Brücken-Ösen-Verbindungspunkten besitzen.

9. Medizinische Folie nach Anspruch 1, 3 oder 8, wobei einige der mehreren amorphen Kreise an einigen der mehreren Brücken angebracht sind.

10. Medizinische Folie nach Anspruch 1, 3 oder 8, wobei die amorphen Kreise abgerundete Ränder besitzen.

11. Medizinische Folie nach einem der vorhergehenden Ansprüche, wobei einige der amorphen Kreise (91) in Brücken (70) angeordnet sind und Ösen zwischen angrenzenden Streifen (52(a)-52(d)).

## Revendications

1. Feuille médicale (50, 55) pour l'implantation dans un patient, la feuille médicale comprenant :
a) un élément plan ayant une épaisseur, des premier et deuxième côtés expansibles (45), des premier et deuxième côtés rétractables (43) et un axe longitudinal (83) s'étendant entre les premier et deuxième côtés rétractables, l'élément plan ayant une première position de zone plus petite pour l'insertion dans le patient, et une deuxième position de zone plus grande pour l'implantation dans le patient ;
b) l'élément plan comprenant une pluralité de bandes adjacentes (51(a)-51(c), 52(a)-52(d)) s'étendant parallèlement les unes aux autres le long de l'axe longitudinal de l'élément plan, les bandes comprenant une pluralité d'entretoises longitudinales (60) et une pluralité de boucles (62) reliant des entretoises adjacentes ;
c) une pluralité de ponts (70) reliant les bandes adjacentes les unes aux autres à des points de connexion pont-à-boucle (72, 74), où les points de connexion pont-à-boucle pour chaque pont sont séparés angulairement relativement à l'axe longitudinal ; et
d) une pluralité de cercles amorphes (91), où quelques-uns des cercles amorphes sont fixés à au moins quelques-unes de la pluralité de boucles le long des premier et deuxième côtés expansibles (45), et quelques-uns de la pluralité des cercles amorphes sont fixés à quelques-unes de la pluralité d'entretoises le long des premier et deuxième côtés rétractables (43) à un point terminal le long de la longueur longitudinale de l'entretoise la plus extérieure dans les bandes adjacentes ; et
où les ponts, boucles et entretoises ont des largueurs (W2, W3, W4) qui sont inférieures à l'épaisseur (T) de l'élément plan.

2. Feuille médicale selon la revendication 1, dans laquelle les boucles comprennent des sections courbées sensiblement semi-circulaires ayant des centres (64), et les points de connexion pont-à-boucle sont décalés des centres des boucles.

3. Feuille médicale selon la revendication 1, dans laquelle l'élément plan est réalisé en un alliage nickel titane super élastique, et où le nombre de points de connexion pont-à-boucle est inférieur au nombre total de boucles sur les bandes.

4. Feuille médicale selon la revendication 3, dans laquelle les boucles comprennent des sections courbées sensiblement semi-circulaires ayant des centres (64), les ponts étant connectés aux boucles à des points de connexion pont-à-boucle ayant des centres (76), les centres des points sont décalés des centres des boucles.

5. Feuille médicale selon la revendication 3, dans laquelle la feuille médicale est réalisée en un alliage comprenant environ 50,5 pour cent à environ 60 pour cent de nickel, et le reste étant du titane.

6. Feuille médicale selon la revendication 5, où la feuille médicale a une température A_{f} entre environ 24° à environ 37° Celsius.

7. Feuille médicale selon la revendication 1 ou 3, dans laquelle au moins quelques-uns de la pluralité de cercles amorphes ont des trous concentriques (93).

8. Feuille médicale selon la revendication 6, dans laquelle les ponts ont un profile courbé non linéaire entre les points de connexion pont-à-boucle.

9. Feuille médicale selon la revendication 1, 3 ou 8, dans laquelle quelques-uns de la pluralité de cercles amorphes sont fixés à quelques-uns de la pluralité de ponts.

10. Feuille médicale selon la revendication 1, 3 ou 8, dans laquelle les cercles amorphes ont des bords arrondis.

11. Feuille médicale selon l'une quelconque des revendications précédentes, dans laquelle quelques-uns des cercles amorphes (91) sont placés dans des ponts (70) et boucles entre des bandes adjacentes (52(a)-52(d)).
